# EUROPEAN PATENT APPLICATION

(11) **EP 2 926 749 A1**
(43) Date of publication of application: **07.10.2015**
(21) Application number: 13858347.1
(22) Date of filing: 27.11.2013
(51) Int. Cl.: A61B 17/66, A61C 8/00

(54) **ALVEOLAR DISTRACTOR**

(30) Priority: 28.11.2012 ES 201231846
(71) Applicant: Mozo-Grau, S.L., 47197 Valladolid (ES)
(72) Inventor: MOZO GRAU, Fernando, E-47197 Valladolid (ES)
(74) Representative: Pereira Toña, Maria Irache
(86) International application number: PCT/ES2013/070821
(87) International publication number: WO 2014/083228

(57) **Abstract**

An alveolar ridge distractor for distracting a bone fragment (2) with respect to a maxillary bone (1) separated by an osteotomy line (11); the distractor is comprised of a threaded rod (5) with an upper piece (3) and a lower piece (4). The threaded rod (5) is formed by a top part (7) where the thread has a specific rotation direction and a bottom part (8) where the rotation direction of the thread is opposite to that of the top part of the rod (7); the upper piece (3) is located at the top of the rod (7) and the lower piece (4) is located at the bottom part of the rod (8); both bodies (3 and 4) have an end part (16) with no external thread, generating a free ring (12) in the area in which both bodies are joined, with no thread. That free ring (12) which is not threaded, is positioned to coincide with the osteotomy line (11).

## Description

### PURPOSE OF THE INVENTION

This invention refers to an alveolar ridge distractor which is used to perform a distraction or contraction between alveolar bone sections. It is used in the treatment of bone disorders and in particular, in the field of oral implantology.

### TECHNICAL PROBLEM THAT MUST BE SOLVED AND BACKGROUND INFORMATION ABOUT THE INVENTION

When a tooth is lost, that loss is accompanied by bone loss, leading to the remodelling of the bone containing the tooth (alveolar bone). That tooth is often replaced by a dental implant which is inserted in site where the tooth used to be (socket) in the alveolar bone that used to support the original tooth.

In oral rehabilitation using an implant, the person receiving the implant must have a bed with sufficient bone volume and height for the implant, to providing adequate stability for the implant. In order to create that bed, after the bone loss, the patient will require an alveolar ridge reconstruction to increase the bone volume in which the implant will subsequently be embedded in the socket.

To perform the alveolar ridge reconstruction, the following methods have normally been used:
- autologous bone graft
- membrane and
- alloplastic materials

Such systems, apart from not giving the expected results, make it necessary to wait for approximately 6 months between each surgical procedure for the ridge to increase in size and the implants to be inserted.

At the present time, an alveolar distraction process is performed, to provide a bone bed that will give stability to the implant. That process is not widely used, due to the complex nature of the surgical technique required.

The alveolar distraction technique allows the height of the alveolar edge to be increased, promoting the formation of new bone and an important increase in the surrounding soft tissue (providing a stable bed for the implant). The technique gives the expected result, with low morbidity and infection rates and reduces the wait time to insert the implants, compared to traditional methods.

To perform the alveolar distraction, a traction force is applied to a portion of the bone, which is shifted vertically to reach the optimum or planned height. The shifting of this fragment leads to the regeneration of the tissues, both the hard tissue (bone) and the soft tissue (gum) during that process.

To insert the device that executes the alveolar distraction, it is necessary to perform an osteotomy (cutting of the bone) to release a bone fragment (plate), leaving a free area (osteotomy line) between the bone fragment and the rest of the jaw.

State of the art alveolar distractors have two parts. One of these parts is fixed to the jaw bone and the other to the released bone fragment released by the osteotomy, leaving one tooth on each side of the osteotomy line. This placement is extremely difficult to achieve and does not only depend on the skill and expertise of the surgeon, as the areas in which the distractor is positioned often present a considerable degree of atrophy and are therefore difficult to access and barely visible, thus making it very difficult to perform this surgery successfully.

Furthermore, the devices known to date have certain limitations when used at low heights, in which the quantity of bone obtained is extremely small.

Several documents exist regarding the state of the art with descriptions of elements used to perform the alveolar distraction technique.

The document that is closest to the state of the art is ES 2 127 134 A1, owned by the author of this application. That document describes a bone distractor that has a central threaded rod and two sleeves which are screwed to the alveolar bone, in a similar way to the one described in this patent application, but which presents the difficult problem of ensuring the accurate positioning of the distractor on both sides of the osteotomy line.

Document WO 2010/061391 A1 shows a bone distractor for increasing the height and width of an alveolar ridge. That distractor consists of an elevation platform for distracting a dynamic segment of the alveolar ridge with respect to a static segment of that ridge, an expansion platform for distracting an oral and/or lingual part of the dynamic ridge section and an element for moving the elevation platform in relation to the static ridge section.

Document US 6 280 191 B1 shows a distractor that is appropriate for permanent placement in the bone, in the form of a distractor that is suitable for an indexed osteotomy following permanent implantation in the bone. The distractor is a means for joining two or more bone sections and for facilitating the movement of a bone section with respect to another bone section.

Document ES 2 162 555 B1 shows an alveolar bone distractor formed by a folding structure that delimits a flat articulated polygonal contour comprised of two parallel supports opposite each other, which are connected by an intermediate screw and two pairs of arms which are symmetrical to the median plane that passes through the screw, and connected to each other and to the supports. One of the arms has an elbow and its free end has a head for fixing to the bone that is to be lengthened.

### DESCRIPTION OF THE INVENTION

This invention refers to an alveolar distractor used to perform a distraction or contraction between jaw bones.

The alveolar distractor described in the invention performs the distraction of a bone fragment with respect to a jaw bone, with the bone fragment and jaw bone being separated by a free space (osteotomy line).

The distractor is formed by a threaded rod with an upper piece and a lower piece. The threaded rod has a top part (in which the thread has a specific rotation direction) and a bottom part (in which the rotation direction of the thread is opposite to the direction of the top part). The top piece of the distractor is located in the upper part of the rod and the bottom piece is at the lower part of the rod.

Both the upper piece and the lower piece of the alveolar distractor forming the subject matter of this invention have an end with no external thread (free ring). Since both pieces are in contact with each other (joined) while the distractor is put in position, they each have a respective area with no external thread in contact, thereby generating a ring with no thread (free ring) at the point where both bodies are joined which is positioned in the patient to coincide with the free area separating the bone fragment from the jaw bone, with the lower piece remaining at one side of the osteotomy line and the upper piece on the other side of that osteotomy line, thereby both bodies to move closer to each other or further away from each other on the rod.

The lower piece of the alveolar distractor is shorter than the upper one, which gives stability to the upper piece in the jaw bone.

The upper part of the alveolar distractor rod has a different sized thread pitch to the one on the lower part of the rod.

The thread pitch in the upper part of the alveolar distractor rod is larger than the thread pitch in the lower part of the rod.

The threaded rod of the alveolar distractor has a hollow at one end, which is used to execute the rotation of the threaded rod by activating a key that in inserted into that hollow.

At least one of the alveolar distractor bodies has a pass-through hole which provides access to the hollow in the rod through that pass-through hole with the bodies on the threaded rod.

One of the bodies of the alveolar distractor has a male connector element and the other piece has a female connector element. These elements are connected to each other inside the bodies when both the bodies are joined. Likewise, the male connector element and the female connector element have a shape that allows the threaded rod to pass through both of them.

### DESCRIPTION OF THE FIGURES

To complete the description, and to allow the characteristics of the invention to be better understood, a set of drawings is attached to this descriptive report, as an inseparable part thereof, which show the following, for illustrative purposes and without limitation:
Figure 1 shows another lateral view of the device forming the subject matter of the invention.
Figure 2 shows a section view of the device forming the subject matter of the invention, configured in the same way as in figure 1.
Figure 3 shows a lateral view of the alveolar distractor in its original state before the alveolar distraction process begins.
Figure 4 shows a lateral view of the alveolar distractor placed in position after the alveolar distraction process.

A list of the different elements shown in the figures included in the invention is given below:
1.- jaw bone
2.- bone fragment
3.- upper piece
4.- lower piece
5.- threaded rod
6.- top part of the rod
7.- bottom part of the rod
8.- part of the rod that is not threaded
9.- hollow in the rod
10.-key
11.-osteotomy line
12.-free ring
13.-pass-through hole
14.-external polygonal shape
16.-end part of the piece
17.-male connector element
18.-female connector element

### DESCRIPTION OF ONE WAY OF USING THE INVENTION

In view of the above, and with reference to the numbering used in the figures, the purpose of the invention is to furnish a device which can be used to perform a distraction or contraction of two bones or bone sections, which is indicated in particular for use in jaw bones (1).

In writing this report, the different invention elements have been designated, with the invention being divided into a top part and a bottom part, based on a geometric criterion and taking as an example a distractor located in the jaw bone (1) of a patient.

The device forming the subject matter of the invention is placed in the patient's jaw bone (1) after performing the osteotomy. That osteotomy is completed, until a bone fragment (2) is released.

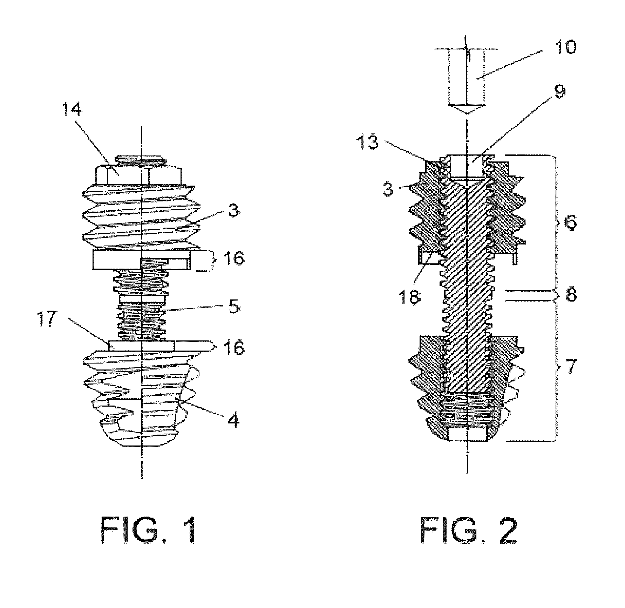

The device forming the subject matter of the invention (as can be observed in figures 1 to 4) is formed by an upper piece (3), a lower piece (4) and threaded rod (5) in which the upper piece (3) and the lower piece (4) are threaded.

Both the upper piece (3) and the lower piece (4) have external threads which are used to fix both the bodies (3 and 4) to the jaw bone (1). The upper piece (3) is inserted into the jaw bone (1) and the lower piece (4) is fixed to the bone fragment (2), with this bone fragment (2) being the part of the bone that will perform the distraction (separate) on those bodies (3and 4) as can be seen in figures 1 to 4). Both the upper piece (3) and the lower piece (4) have an external area (16) with no thread, so that once the lower piece (4) and the upper piece (3) are installed on the rod (5), with the end areas (16) of both bodies in contact, a free ring (12) with no thread is created in the area where both bodies (3 and 4) touch each other. The free ring with no thread (12) drastically reduces the errors and complications that may arise in placing the bodies (3 and 4) opposite each other, on each side of the osteotomy line (11), as is done during surgery to position the distractor in the bone.

The lower piece (4) and the upper piece (3) have a female connector element (18) and a male connector element (17) which act when the two bodies (3 and 4) are jointed, thus preventing the dismantling of the distractor when it is inserted into the jaw bone (1).

The position of the male connector element (17) and the female connector element (18) in the upper piece (3) and in the lower piece (4), is not restricted in any way, as it makes no difference whether the upper piece (3) contains the male connector element (17) and the lower piece (4) the female connector element (18) or whether on the contrary, the upper piece (3) contains the female connector (18) and the lower one (4) the male connector (17).

The upper piece (3) is longer than the lower piece (4), which improves its stability during the installation of the bone fragment (2), thus preventing the upper piece from losing primary stability in the alveolar fragment.

The threaded rod (5) of the device forming the subject matter of the invention is divided into three parts, a top part (6) with a thread in one direction, a bottom part (7) with a thread in the opposite direction to the thread of the top part (6) and a central part between them, with no thread (8). This division of the rod into two parts (6 and 7) with opposing rotation directions means that the rotation of the rod (5) in one direction brings both the bodies closer to each other (3 and 4), while its rotation in the opposite directions causes the bodies to move further away from each other (3 and 4).

In the distraction movement of the bodies (3 and 4), the lower piece (4) is fixed to the jaw bone (1), meaning that it does not move, while the upper piece (3) moves away from the lower piece (4). During this distraction, the upper piece (3) drags the bone fragment (2) to which it is fixed, thus achieving the purpose of the device, which is to obtain an optimum alveolar bone height (1) for inserting the implant.

Both the threaded parts (6 and 7) of the rod (5) have different sized thread pitches, and the thread pitch of the upper part of the rod (5) is larger than that of the bottom part of the rod (7). This difference between both thread pitches leads to an increase in the length of the distractor run and the total resulting height of the distracted bone is increased by the same size as the height of the distractor.

At the top part of the rod (5) is a hollow (9) (shown in figure 2), and when a key (10) is inserted into that hollow (9), when that key is activated (10), the rod (5) rotates in one direction, for instance to the right.

The top part (3) has a pass-through hole (13) (shown in figure 2) and the rod (5) passes through it at the end with the hollow (9), leaving that hollow (9) exposed, so that the key (10) can be placed in that hollow (9) in order to rotate the rod (5). The upper piece (3) of the device has a polygonal shape (14) around the pass-through hole (13) at the end of the upper piece (3) next to the hollow (9) in the rod (5).

The device forming the subject matter of the invention operates based on a procedure similar to the one explained below:
- an osteotomy is performed in the jaw bone (1), to release a bone fragment (2), and the osteotomy line (11) is defined, which is a free space (without bone);
- the alveolar distractor is positioned with the upper piece (3) in the jaw bone and the lower piece (4) fixed to the bone fragment (2), and in this position, the free ring (12) with no thread generated by joint both the bodies (3 and 4) is positioned opposite the osteotomy line (11);

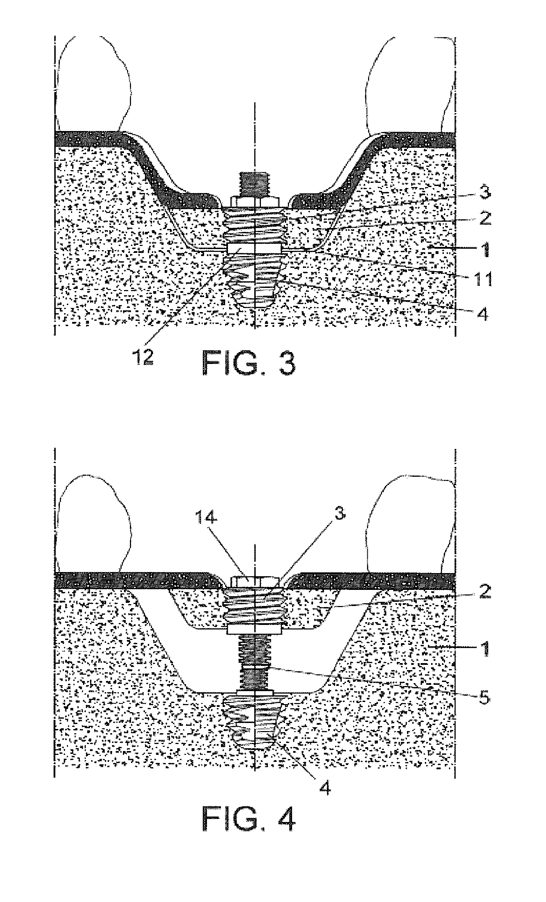

- the distraction of the bone fragment (2) with respect to the jaw bone (1) commences, transmitting a rotation movement to the rod (5) through a key (10) and by means of this rotation of the rod (5), the upper part of the rod (7) is extracted from the lower part (4) and the upper part (3) moves further away from the lower piece (4) on the rod (5);
- the rod (5) continues to rotate until the distance between both bodies (3 and 4) provides the optimum height for positioning an implant (show in figure 4), with the interval for applying each rotation of the rod (5) being different, depending on each type of treatment;
- after completing the distraction process and in accordance with the established time, the distractor is removed and the implant is put in place.

The invention is not limited to the specific use described in this document. Experts in the subject may develop other uses, in the light of the description given herein. Consequently, the scope of this invention is defined by the following claims.

## Claims

1. AN ALVEOLAR DISTRACTOR for performing a distraction on a bone fragment (2) with respect to a jaw bone (1), separated by an osteotomy line (11); that distractor is comprised of:
- a threaded rod (5), divided into a top part (6) and a bottom part (7) in which both parts (6 and 7) have a rod with opposing rotation directions,
- an upper piece (3) located at the top part (6) of the rod (5),
- a lower piece (4) located at the bottom part (7) of the rod (5), **characterised in that** the upper piece (3) and the lower piece (4) have an external area(16) with no thread, thereby generating a free ring (12) with no thread in the site where both the bodies (3 and 4) are joined, and that ring (12) with no thread is positioned to coincide with the osteotomy line (11).

2. AN ALVEOLAR DISTRACTOR, as set out in claim 1, **characterised in that** the lower piece (4) is shorter than the upper piece (3), providing stability to the upper piece (4) inside the alveolar bone (1).
15

3. AN ALVEOLAR DISTRACTOR, as set out in both the above claims, **characterised in that** the top part (6) of the rod (5) has a thread pitch that is different from that of the lower part (7) of the rod (5).

4. AN ALVEOLAR DISTRACTOR, as set out in claim 3, **characterised in that** the thread pitch of the top part (6) of the rod (5) is larger than the thread pitch of the bottom part (7) of the rod (5).

5. AN ALVEOLAR DISTRACTOR, as set out in claim 1, **characterised in that** the threaded rod (5) has a hollow (9) at one end, through which the threaded rod can rotate (5), on activating a key (10) inserted into that hollow (9).

6. AN ALVEOLAR DISTRACTOR, as set out in either of claims 1 and 2, **characterised in that** at least one of the pieces (3 and 4) has a pass-through hole (13) which gives access to the hollow (9) in the rod through that pass-through hole (13) when the pieces (3 and 4) are placed on the threaded rod (5).

7. AN ALVEOLAR DISTRACTOR, as set out in either of claims 1 and 2, **characterised in that** one of the pieces (3 and 4) has a male connector element (17) and the other piece (3 and 4) has a female connector element (18) which are connected inside both pieces (3 and 4) when they are joined. Furthermore, the male connector element (17) and the female connector element (18) have a shape that allows the threaded rod (5) to pass through them.
